# EUROPEAN PATENT SPECIFICATION

(11) **EP 2 712 563 B1**
(45) Date of publication and mention of the grant of the patent: **13.09.2017**
(21) Application number: 13186873.9
(22) Date of filing: 01.10.2013
(51) Int. Cl.: A61B 17/72, A61B 17/86

(54) **Reversible bone coupling device**
Reversible Knochenkupplungsvorrichtung
Dispositif de couplage osseux réversible

(30) Priority: 01.10.2012 US 201213632337
(43) Date of publication of application: 02.04.2014
(60) Divisional application: 17185419.3
(73) Proprietor: Nextremity Solutions, Inc., Warsaw, Indiana 46580 (US)
(72) Inventor: Weiner, Lon S., Rumsen, New Jersey 07760 (US); Katchis, Stuart D., Scarsdale, New York 10583 (US); Alfaro, Arthur A., Colts Neck, New Jersey 07722 (US); Van Iperen, Willem H.P., Westfield, New Jersey 07090 (US); Pepper, John R., Cheshire, Connecticut 06410 (US); Truman, Mari S., Warsaw, Indiana 46580 (US)
(74) Representative: Buzzi, Franco

(56) References cited:
- US-A- 5 417 692
- US-A1- 2010 036 439
- US-A1- 2011 004 255
- US-A1- 2011 082 508
- US-B1- 6 458 134

## Description

### TECHNICAL FIELD

The present disclosure relates to devices and methods for coupling bones with reversibly engaging bone coupling devices.

### RELATED ART

Hammertoe deformity, the most common deformity of the lesser toes, is a flexion deformity of the proximal interphalangeal (PIP) joint of the toe, with hyperextension of the metatarsophalangeal (MTP) and distal interphalangeal (DIP) joints. Progressive PIP joint flexion deformity typically leads to compensatory hyperextension of the MTP and DIP joints. This makes the PIP joint prominent dorsally. Pain occurs due to rubbing of the prominence against the patient's shoe. The deformity is flexible at first but usually becomes fixed over time. When the deformity is flexible, various procedures can be utilized that involve manipulation of the involved tendons. However, when the deformity is fixed, PIP fusion or joint replacement is often required. Implants available for this purpose include the Weil-Carver™ Hammertoe Implant (Biomet®, Inc., Warsaw, IN), Flexible Digital Implant (Tornier, Inc. Edina, MN), SHIP Implant (Sgarlato Labs, Campbell CA), Digital Compression Screw (BioPro®, Port Huron MI), Smart Toe™ Intramedullary Memory Implant (Memometal Inc., Memphis TN), StayFuse™ Intramedullary Fusion Device (Tornier, Inc. Edina, MN), and Pro-Toe (Wright Medical, Arlington TN). The latter three implants are used when fusion is desired, since the other implants allow some flexibility of the joint. With all current implants, placement is critical because, when mounted, there is no adjustability following initial implantation in the angle of flexion between the two cut bones to be coupled.

From US-2011/004255 in the name of the same Applicant a reversible bone coupling device is known, which corresponds to the pre-characterizing part of claim 1.

There is a need for alternative designs for implants for coupling two bone pieces, including implants that reversibly fix the two bone pieces. The present inventive concept addresses that need.

### SUMMARY

The reversible bone coupling device of the invention is set forth in claim 1. Preferred embodiments are defined in the dependent claims.

Additional aspects, advantages, and utilities of the present disclosure will be set forth in part in the description which follows and, in part, will be obvious from the description, or may be learned by practice of the embodiments of the present disclosure.

### BRIEF DESCRIPTION OF THE DRAWINGS

Only the reversible bone coupling device shown in Figs. 1-10 is an embodiment of the invention, whereas the reversible bone coupling devices shown in Figs. 11-26 do not fall within the scope of the claims.
FIG. 1 illustrates a bone fusion device in accordance with an embodiment of the present disclosure.
FIG. 2 illustrates a bone fusion device after fusion of the male and female component in accordance with an embodiment of the present disclosure.
FIG. 3 illustrates a side view of a male component of a bone fusion device in accordance with an embodiment of the present disclosure.
FIG. 4 illustrates another side view of a male component of a bone fusion device in accordance with an embodiment of the present disclosure.
FIG. 5 illustrates a perspective view of a male component of a bone fusion device in accordance with an embodiment of the present disclosure.
FIG. 6 illustrates a perspective and cut-away view of a female component of a bone fusion device in accordance with an embodiment of the present disclosure.
FIG. 7 illustrates a view of the opening of a cavity of a female component of a bone fusion device in accordance with an embodiment of the present disclosure.
FIGS. 8a-c illustrate perspective views of a ring of a reversibly engaging locking mechanism of a bone fusion device in accordance with an embodiment of the present disclosure.
FIG. 9 illustrates a view of the opening of a cavity of a female component of a bone fusion device with a partially disengaged ring of a reversibly engaging locking mechanism in accordance with an embodiment of the present disclosure.
FIG. 10 illustrates a perspective view of a female component of a bone fusion device including an opening for receiving a ring of a reversibly engaging locking mechanism.
FIG. 11 illustrates a bone fusion device in accordance with an embodiment of the present disclosure.
FIG. 12 illustrates a male component of a bone fusion device in accordance with an embodiment of the present disclosure.
FIG. 13 illustrates another male component of a bone fusion device in accordance with an embodiment of the present disclosure.
FIG. 14 illustrates a female component of a bone fusion device in accordance with an embodiment of the present disclosure.
FIG. 15 illustrates a cross-sectional view of a bone fusion device after coupling of a female component and a male component in accordance with an embodiment of the present disclosure.
FIG. 16 illustrates a bone fusion device after fusion of a female component and a male component in accordance with an embodiment of the present disclosure.
FIG. 17 illustrates a view into a cavity of a female component of a bone fusion device in accordance with an embodiment of the present disclosure.
FIG. 18 illustrates a perspective view of a female component of a bone fusion device in accordance with an embodiment of the present disclosure.
FIG. 19 illustrates a cross-sectional view of a female component of a bone fusion device in accordance with an embodiment of the present disclosure.
FIG. 20 illustrates another integral deflection means comprising an inline deformed tab of a reversibly engaging locking mechanism for locking with a groove of a connector of a male component in accordance with an embodiment of the present disclosure.
FIG. 21 illustrates a bone fusion device in accordance with an embodiment of the present disclosure.
FIG. 22 illustrates a female component of a bone fusion device in accordance with an embodiment of the present disclosure.
FIG. 23 illustrates a cross-sectional view of a female component of a bone fusion device in accordance with an embodiment of the present disclosure.
FIG. 24 illustrates a cross-sectional view of a bone fusion device where the male component has been inserted into the female component, in accordance with an embodiment of the present disclosure.
FIG. 25 illustrates an elevated magnified perspective view illustrating a ring, in accordance with an embodiment of the present disclosure.
FIG. 26 illustrates an elevated side view perspective illustrating a male component, in accordance with an embodiment of the present disclosure.

### DETAILED DESCRIPTION

The present disclosure relates to devices and methods for coupling bones with reversibly engaging bone coupling devices. In some embodiments, a reversible bone coupling device facilitates adjustment of an angle between two bones to be coupleed, as well as being reversibly engaging to assist in corrections of the coupling of the two bones.

The reversible bone coupling device comprises a first component and a second component. The first component comprises a first elongated stem portion comprising a first end and a first top opposite the first end. The first elongated stem portion is suitable for insertion from the first end longitudinally into a surface of a first bone piece of a bone. The second component comprises a second elongated stem portion comprising a second end and a second top. The second elongated stem portion is suitable for insertion from the second end longitudinally into a surface of a second bone piece of the bone. The second component further comprises a connector extending from the second top. The connector is capable of coupling with the first component and locking therewith to couple the first component and the second component. The first component further comprises a reversibly engaging locking mechanism that locks the connector, and facilitates unlocking of the connector to allow adjustment of the fusion between the first component and the second component.

In certain embodiments, the first component is a female component and the second component is a male component. The first elongated stem portion of the female component may comprise an opening that extends axially from the first top toward the first end. The connector may comprise an elongated shaft, a proximal end, a top of shaft near the proximal end, and a distal end, where the connector is capable of insertion into the opening in the first elongated stem portion and locking therein to couple the male component and the female component.

The device is useful for coupling any two bone pieces, and by way of the reversibly engaging mechanism, the connector may be reversed to adjust the position of the connector or remove the connector entirely in situations where adjustment of the device may be necessary or contemplated to further assist in coupling or fusing cut surfaces of bones. As a result, the device is adaptable to any bone size, shape, or configuration of any patient. In some embodiments, the device is particularly useful in coupling or fusing cut surfaces of bones such as cut ends of fingers or toes. This may facilitate the treatment of hammertoe, claw toe, mallet toe, or curly toe. In these embodiments, the first elongated stem portion is suitable for insertion from the first end longitudinally into a cut surface of a resected phalanx, metatarsal or metacarpal, or a cut diaphyhsis, and the second elongated stem portion is suitable for insertion from the second end longitudinally into a cut surface of a resected phalanx, metatarsal or metacarpal, or a cut diaphyhsis.

In the various embodiments described herein and corresponding with the Figures provided herewith, a bone fixation or fusion method and system are described. A first component of a bone fusion device is inserted into a first bone piece. A second component of a bone fusion device is inserted into a second bone piece. A connector of the second component is inserted into a cavity of the first component. The connector is locked within the cavity of the first component by a reversibly engaging locking mechanism to facilitate formation of a fused bone. If necessary, the reversibly engaging locking mechanism may be adjusted to allow the connector and corresponding second component to be longitudinally adjusted or removed from the first component to facilitate adjustments or modifications during or after surgery.

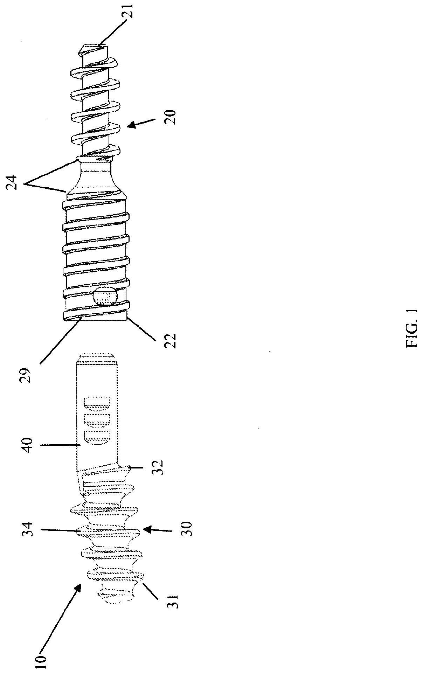

FIG. 1 illustrates a bone fusion device in accordance with an embodiment of the present disclosure. Bone fusion device 10 includes a female component 20 and a male component 30. Female component 20 is an elongated stem comprising a first end 21, a first top 22, and a cavity 29. Female component 20 also includes a spiraling thread 24 on the exterior, suitable for screwing female component 20 into a bone or bone piece.

Male component 30 is an elongated stem comprising a second end 31 and a second top 32. Male component 30 further includes a connector 40 extending from the second top 32. In certain embodiments, connector 40 may be configured to be adjustably attached to the elongated stem of the male component 30, thus supporting the effective lengthening or shortening of the male component 30. Male component 30 also includes a spiraling thread 34 on the exterior, suitable for screwing male component 30 into a bone or bone piece.

Female component 20 and male component 30 can independently be cylindrical or conical, or any combination thereof. Where the illustrated embodiments show spiraling threads as means to anchor a male component or female component to a bone, alternate anchoring means may be used. Where present, the spiraling threads can be of any type known in the art for screwing into a bone. Thus, in some embodiments, the spiraling thread is a continuous spiraling thread. In other embodiments, the spiraling thread allows self-tapping and/or self-threading.

In an embodiment, the spiraling threads may be continuous. In yet another embodiment, the spiraling threads may spiral in the same direction so that when the device is screwed into opposing bone surfaces and coupled, the opposing pitch of the threads in the bone prevents the device from unscrewing.

The embodiments described herein are not limited to any particular pitch of one rotation of the continuous spiraling thread. For example, the pitch may be 5 mm or greater, 4 mm, 3 mm, 2 mm, 1 mm, or any distance in between the aforementioned distances.

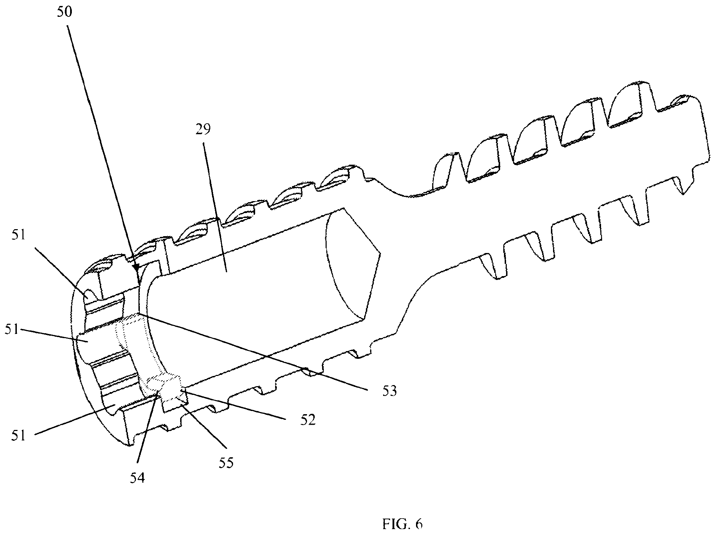

Connector 40, as shown in FIG. 1, may comprise grooves for interlocking with a reversibly engaging locking mechanism of female component 20. When a particular groove engages reversibly engaging locking mechanism 50 (as shown in Fig. 6), reversibly engaging locking mechanism locks the connector of the male component within the female component such that the bone pieces that the male component and the female component are screwed into become coupled or fused as a bone fusion.

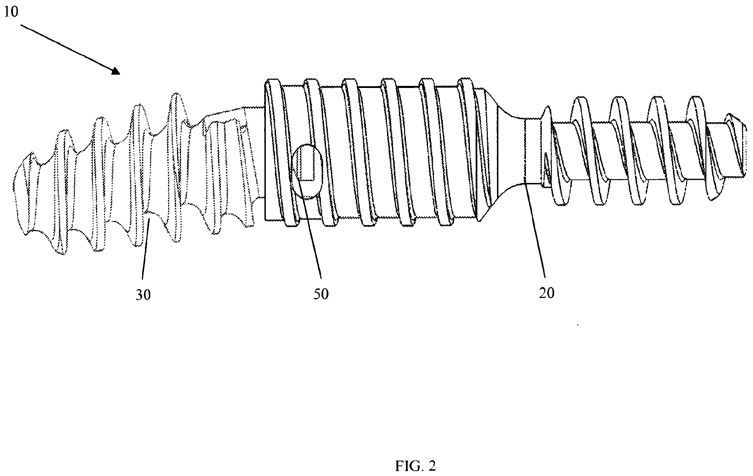

FIG. 2 illustrates a bone fusion device after coupling of the male and female component in accordance with an embodiment of the present disclosure. FIG. 2 shows bone fusion device 10 after connector 40 of male component 30 has been inserted into cavity 29 of female component 20. When bone fusion device 10 is in the state as depicted, the bones that male component 30 and female component 20 have been screwed into are effectively coupled or fused together. Connector 40 is held and locked within the female component by the reversibly engaging locking mechanism 50.

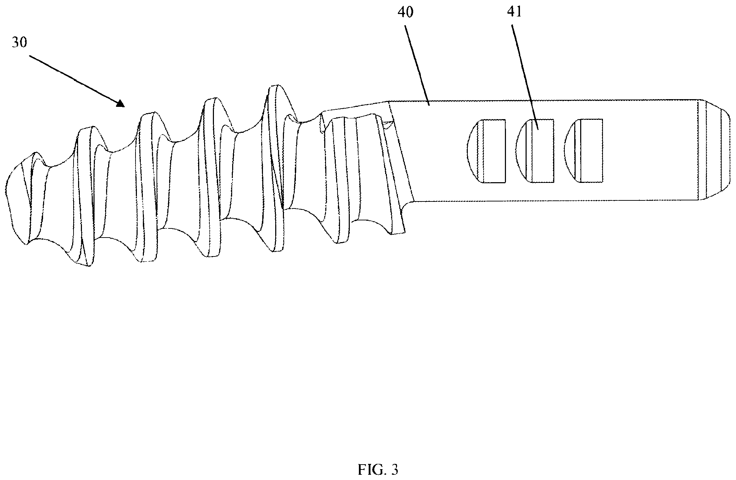

FIG. 3 illustrates a side view of a male component of a bone fusion device in accordance with an embodiment of the present disclosure. Male component 30, as shown, includes connector 40, which comprises a plurality of grooves 41 for engaging with a reversibly engaging locking mechanism to lock connector 40 within a female component to couple the male component and the female component.

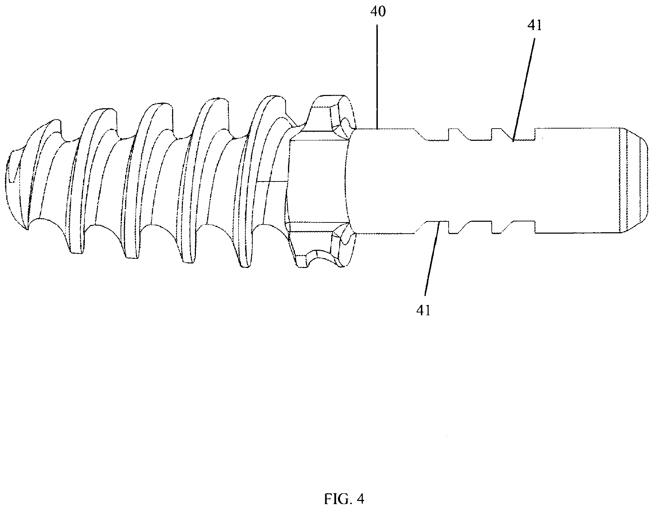

FIG. 4 illustrates another side view of a male component of a bone fusion device in accordance with an embodiment of the present disclosure. The plurality of grooves 41, as shown in FIG. 4, may be on two sides of connector 40.

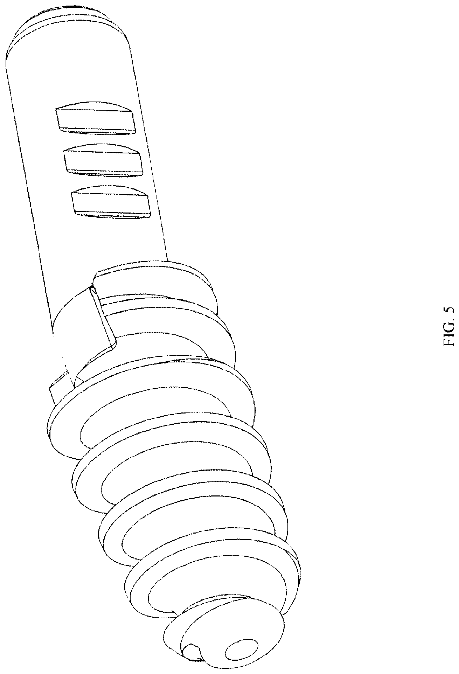

FIG. 5 illustrates a perspective view of a male component of a bone fusion device in accordance with an embodiment of the present disclosure.

FIG. 6 illustrates a perspective and cut-away view of a female component of a bone fusion device in accordance with an embodiment of the present disclosure. In the cross-section view, the inside of cavity 29 is viewable. Within cavity 29 are a plurality of lobes 51 and a ring 52, both of which are a part of reversibly engaging locking mechanism 50. Ring 52 rests within a slot 55 that is sized for receiving ring 52. In an embodiment, the plurality of lobes 51 is located at the opening of cavity 29. The plurality of lobes 51 are configured such that any one or two of the lobes may receive a corresponding boss 53 of ring 52 to prevent rotation of ring 52. As FIG. 6 illustrates a cut-away view, only three lobes 51 are viewable and one boss 53 of ring 52. Additionally, a portion of a chamfer 54 of ring 52 is viewable. Chamfer 54 protrudes so that when connector 40 is inserted into cavity 29, chamfer 54 engages with any of grooves 41 to lock connector 40 within female component 20. Ring 52 allows rotational unlocking of connector 40 from female component 20. Ring 52 may be rotated to unlock chamfer 54 from a groove of connector 40, and allow connector 40 to be pulled outwards from cavity 29. This facilitates complete removal of connector 40 from cavity 29 or adjustment such that a different groove of connector 40 can be caused to engage with chamfer 54 to lock connector 40 within female component 20 in a new position.

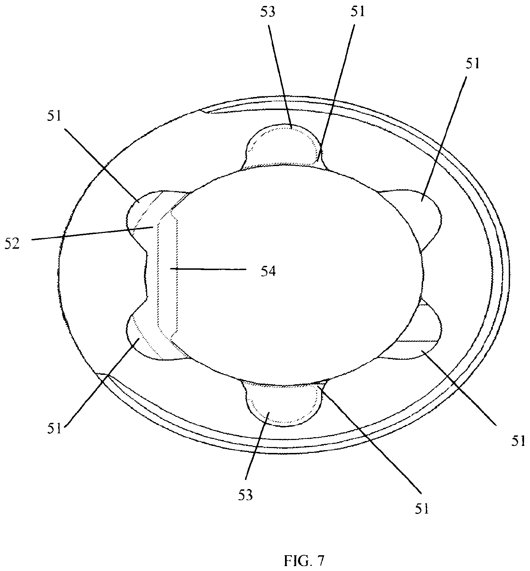

FIG. 7 illustrates a view of the opening of a cavity of a female component of a bone fusion device looking from the first end 21 toward the first top 22, in accordance with an embodiment of the present disclosure. Cavity 29, as shown, includes six lobes 51, and ring 52 which rests within a slot within the cavity. Ring 52 includes two bosses 53, each of which is engaged with a corresponding lobe 51 to hold ring 52 in place within cavity 29. More specifically, lobes 51 anchor bosses 53 of a ring 52 to prevent rotation of ring 52. Chamfer 54, shown in FIG. 7, protrudes from ring 52 in order to engage with grooves of a connector. The cavity may include any number of lobes depending on the size of the lobes and the cavity, provided that the lobes of sufficient size to secure two or more bosses to prevent rotation of the ring. The ring may have two or more bosses provided that they are of sufficient size to remain secured in the lobes so as to prevent rotation of the ring.

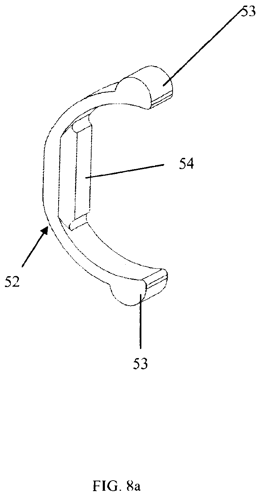

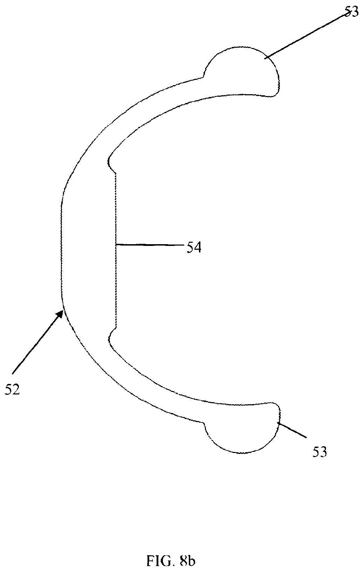

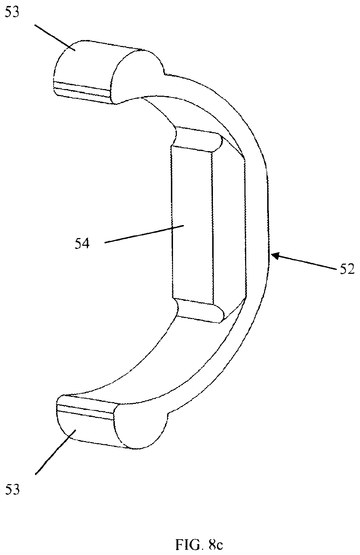

FIGS. 8a-c illustrate perspective views of a ring of a reversibly engaging locking mechanism of a bone fusion device in accordance with an embodiment of the present disclosure.

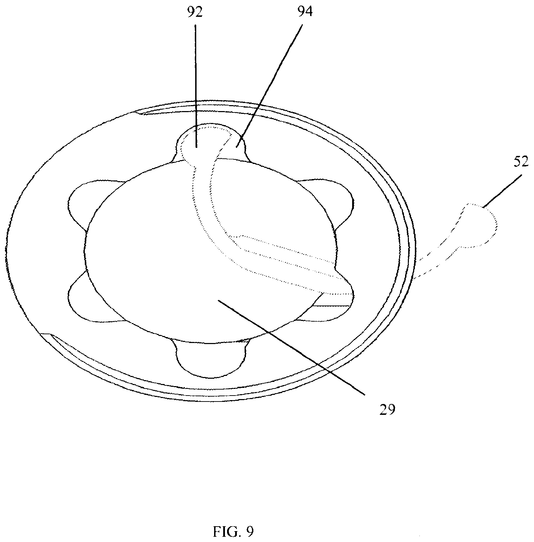

FIG. 9 illustrates a view of the opening of a cavity 29 of a female component of a bone fusion device with a partially disengaged ring 52 of a reversibly engaging locking mechanism in accordance with an embodiment of the present disclosure. Ring 52 as shown is partially disengaged since only one boss 92 is currently engaged with a lobe 94. FIG. 9 is meant to illustrate how ring 52 is inserted into cavity 29. Ring 52 is inserted into cavity 29 through an opening (not shown) on a side of female component 20. Once one end of ring 52 is inserted, a boss, such as boss 92 of that end may be engaged with a lobe 94.

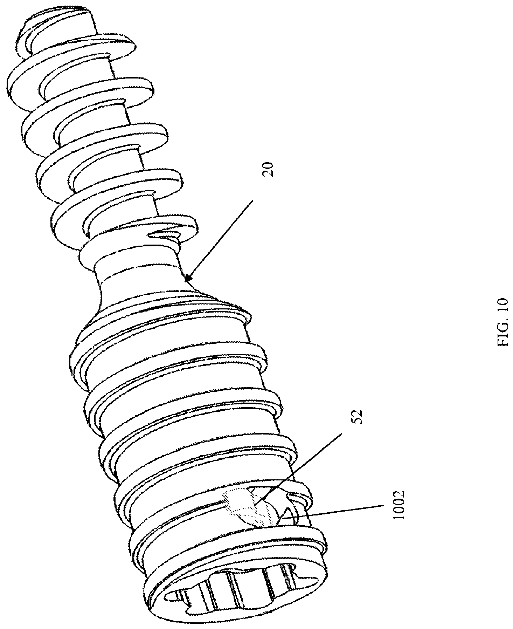

FIG. 10 illustrates a perspective view of a female component of a bone fusion device including an opening for receiving a ring of a reversibly engaging locking mechanism. FIG. 10 shows an opening 1002 of female component 20 for receiving ring 52.

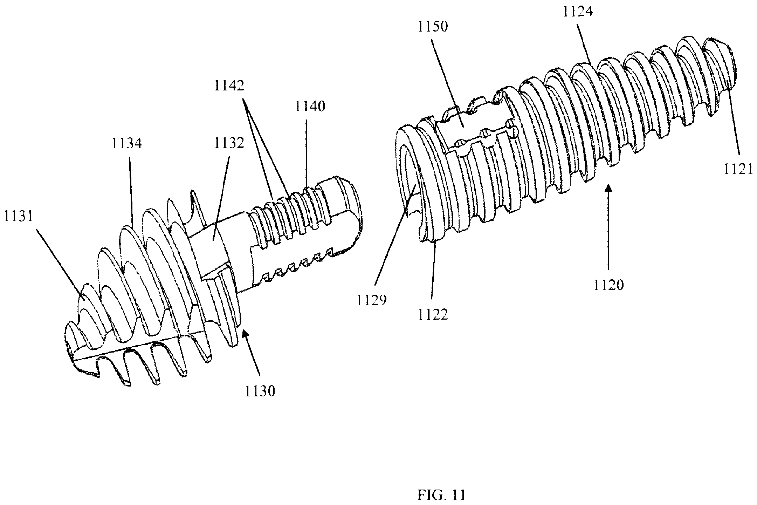

FIG. 11 illustrates a bone fusion device in accordance with an embodiment of the present disclosure. Bone fusion device 1110 includes a female component 1120 and a male component 1130. Female component 1120 is an elongated stem comprising a first end 1121, a first top 1122, and a cavity 1129. Female component 1120 also includes a spiraling thread 1124 on the exterior, suitable for screwing female component 1120 into a bone or bone piece.

Male component 1130 is an elongated stem comprising a second end 1131 and a second top 1132. Male component 1130 further includes a connector 1140 extending from second top 1132. Male component 1130 also includes a spiraling thread 1134 on the exterior, suitable for screwing male component 1130 into a bone or bone piece.

Female component 1120 and male component 1130 can independently be cylindrical or conical, or any combination thereof. Where the illustrated embodiments show spiraling threads as means to anchor a male component or female component to a bone, alternate anchoring means may be used. Where present, the spiraling threads can be of any type known in the art for screwing into a bone. Thus, in some embodiments, the spiraling thread is a continuous spiraling thread. In other embodiments, the spiraling thread allows self-tapping and/or self-threading.

In an embodiment, the spiraling threads may be continuous. In yet another embodiment, the spiraling threads may spiral in the same direction so that when the device is screwed into opposing bone surfaces and coupled, the opposing pitch of the threads in the bone prevents the device from unscrewing.

The embodiments described herein are not limited to any particular pitch of one rotation of the continuous spiraling thread. For example, the pitch may be 5 mm or greater, 4 mm, 3 mm, 2 mm, 1 mm, or any distance in between the aforementioned distances.

Connector 1140, as shown in FIG. 11, may comprise grooves 1142 for interlocking with a reversibly engaging locking mechanism 1150 of female component 1120. When a particular groove 1142 engages reversibly engaging locking mechanism 1150, reversibly engaging locking mechanism 1150 locks the connector of the male component within the female component such that the bone pieces that the male component and the female component are screwed into become coupled or fused as a bone fusion. Reversibly engaging locking mechanism 1150 may be an integral deflection means such as an integral tab that has been cut into a wall of female component 1120. The integral tab may be deflected inward or be a machined tab protruding inside the main diameter of cavity 1129. The integral tab fits within grooves of connector 1140 in order to lock connector 1140 in place within female component 1120. Axial force applied to the integral tab will unlock the integral tab from a groove of connector 1140. For example, integral tab may be bent inwards or outwards at a specific angle to release connector 1140. Once released, connector 1140 may be adjusted within cavity 1129 of female component 1120, or connector 1140 may be removed entirely.

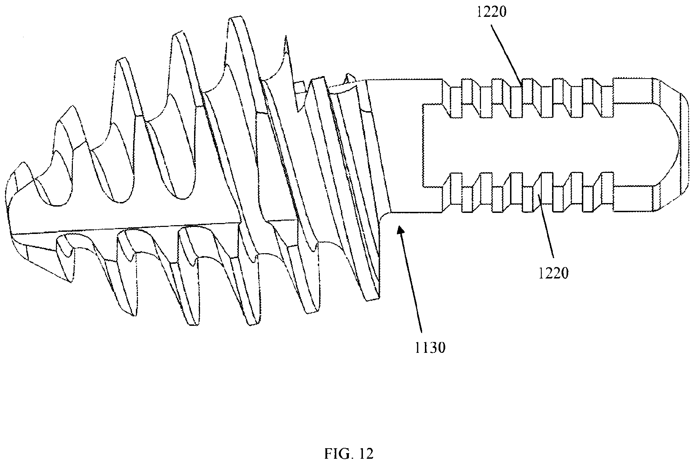

FIG. 12 illustrates a male component of a bone fusion device in accordance with an embodiment of the present disclosure. Male component 1130, as shown, includes a plurality of grooves 1220. The plurality of grooves 1220 are configured as segmental recesses, which support rotational unlocking of connector 1140 from reversibly engaging locking mechanism 1150 which comprises an integral tab.

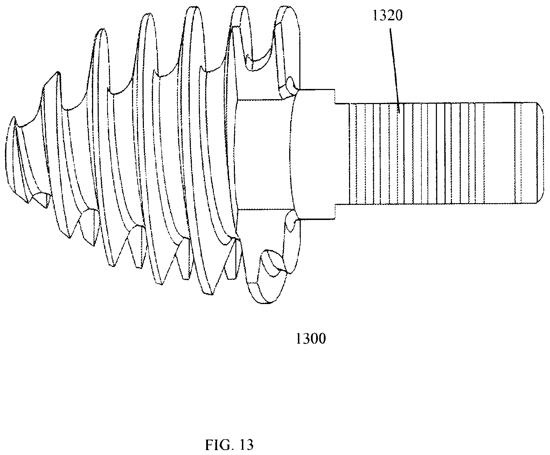

FIG. 13 illustrates another male component of a bone fusion device in accordance with an embodiment of the present disclosure. Male component 1300 includes a plurality of grooves 1320. The plurality of grooves 1320 are full radial grooves that may lock with an integral tab of reversibly engaging locking mechanism, and unlock when axial force is applied to reversibly engaging locking mechanism.

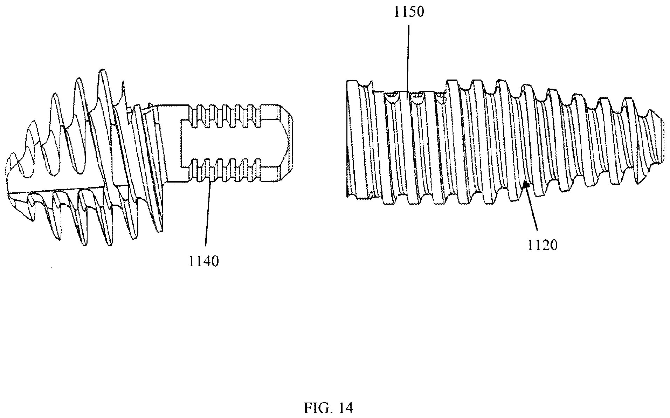

FIG. 14 illustrates a female component of a bone fusion device in accordance with an embodiment of the present disclosure. Female component 1120, is shown. On a wall of female component 1120, is an integral tab of reversibly engaging locking mechanism 1150, which has been cut into the wall of female component 1120.

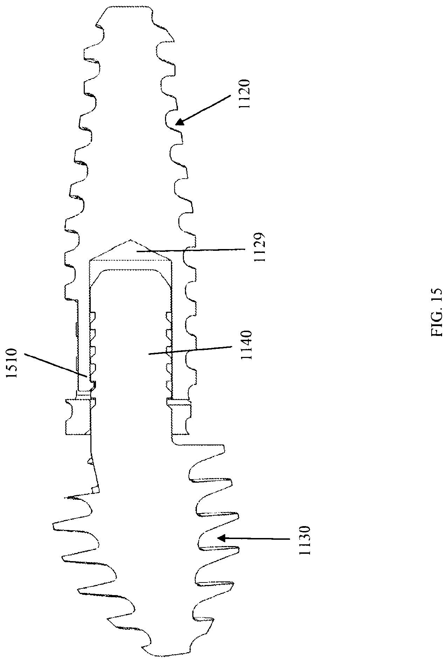

FIG. 15 illustrates a cross-sectional view of a bone fusion device after coupling of a female component and a male component in accordance with an embodiment of the present disclosure. Connector 1140 of male component 1130 has been inserted into cavity 1129 of female component 1120. Connector 1140 is held in place by integral tab 1510 of the reversibly engaging locking mechanism. Integral tab 1510 may be deflected inwards or protrude inside the main diameter of the cavity 1129. Application of axial force to integral tab 1510 facilitates unlocking of integral tab 1510 from a groove of connector 1140 to allow for adjustment of connector 1140 within cavity 1129.

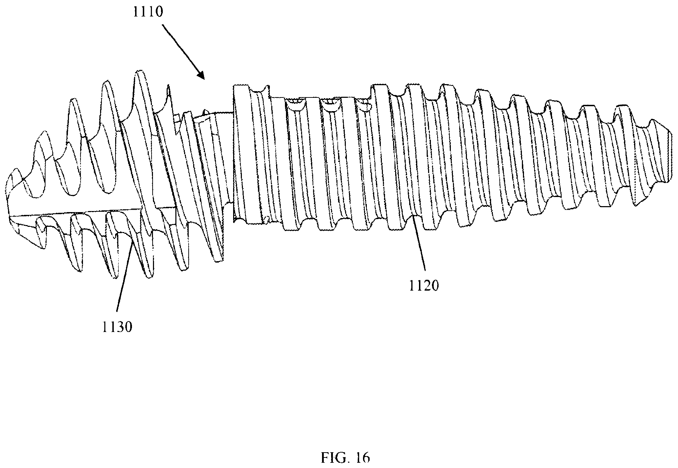

FIG. 16 illustrates a bone fusion device after coupling of a female component and a male component in accordance with an embodiment of the present disclosure. Bone fusion device 1110 is shown by FIG. 16 in a position where both female component 1120 and male component 1130 are coupled.

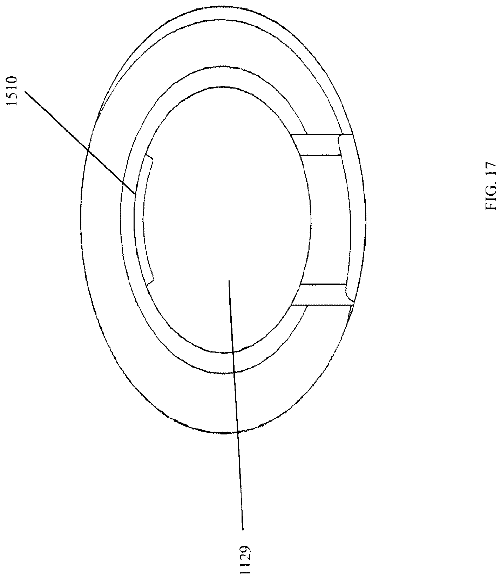

FIG. 17 illustrates a view into a cavity of a female component of a bone fusion device in accordance with an embodiment of the present disclosure. In the view into cavity 1129 provided by FIG. 17, a portion of integral tab 1510 is visible. This portion of integral tab 1510 of the reversibly engaging locking mechanism is a portion that engages with a groove of the connector to lock the connector in place within cavity 1129.

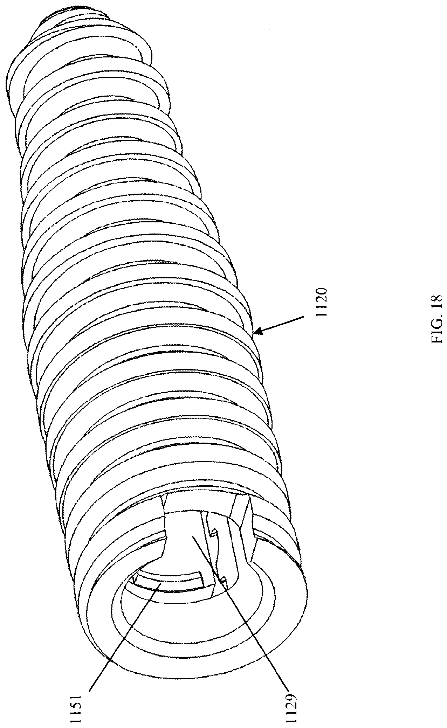

FIG. 18 illustrates a perspective view of a female component of a bone fusion device in accordance with an embodiment of the present disclosure. FIG. 18 provides a view of integral tab 1510 within cavity 1129 of female component 1120.

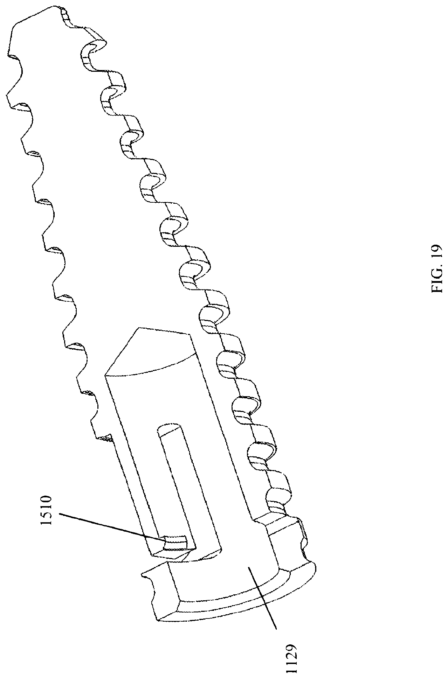

FIG. 19 illustrates a cross-sectional view of a female component of a bone fusion device in accordance with an embodiment of the present disclosure. The cross-sectional view provides a view of an inside of cavity 1129, as well as a full view of integral tab 1510 of a reversibly engaging locking mechanism, which is used to hold a connector in place within cavity 1129.

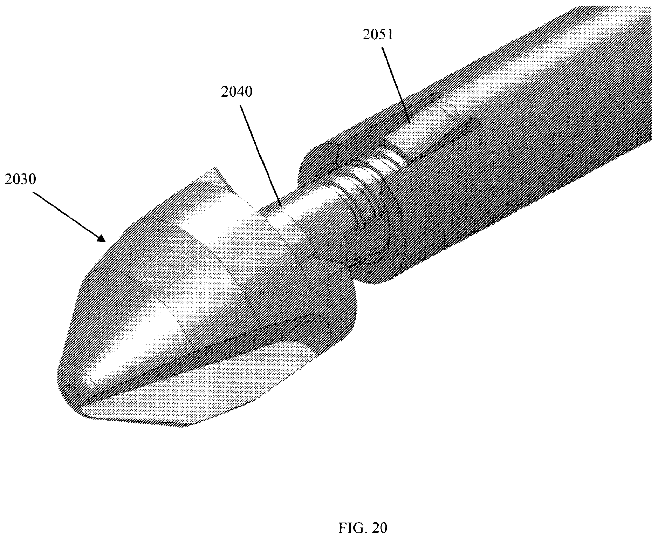

FIG. 20 illustrates another integral deflection means comprising an inline deformed tab of a reversibly engaging locking mechanism for locking with a groove of a connector of a male component in accordance with an embodiment of the present disclosure. Male component 2030 is in a form of a conical shape, with a connector 2040 including a plurality of grooves. Each of the grooves of connector 2040 may engage with a reversibly engaging locking mechanism of a female component 2020 of bone fusion device 2010. Reversibly engaging locking mechanism includes an inline deformed tab 2051 for engaging with grooves of connector 2040. Inline deformed tab 2051 may be bent inwards, and due to a combination of driving slot and tab, may result in easier manufacture.

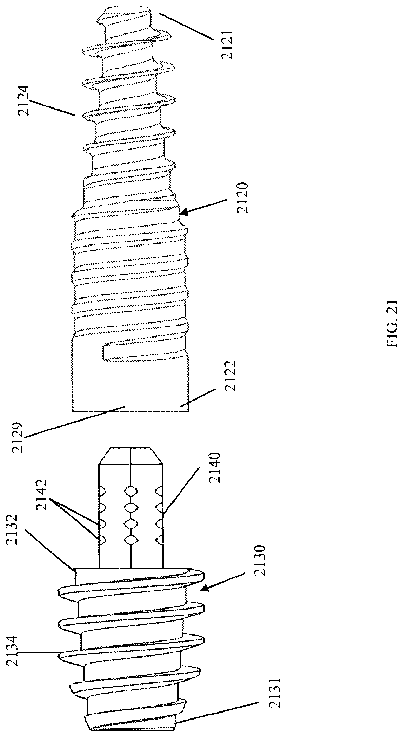

FIG. 21 illustrates a bone fusion device in accordance with an embodiment of the present disclosure. Bone fusion device 2110 includes a female component 2120 and a male component 2130. Female component 2120 is an elongated stem comprising a first end 2121, a first top 2122, and a cavity 2129. Female component 2120 also includes a spiraling thread 2124 on the exterior, suitable for screwing female component 2120 into a bone or bone piece.

Male component 2130 is an elongated stem comprising a second end 2131 and a second top 2132. Male component 2130 further includes a connector 2140 extending from second top 2132. Male component 2130 also includes a spiraling thread 2134 on the exterior, suitable for screwing male component 2130 into a bone or bone piece.

Female component 2120 and male component 2130 can independently be cylindrical or conical, or any combination thereof. Where the illustrated embodiments show spiraling threads as means to anchor a male component or female component to a bone, alternate anchoring means may be used. Where present, the spiraling threads can be of any type known in the art for screwing into a bone. Thus, in some embodiments, the spiraling thread is a continuous spiraling thread. In other embodiments, the spiraling thread allows self-tapping and/or self-threading.

In an embodiment, the spiraling threads may be continuous. In yet another embodiment, the spiraling threads may spiral in the same direction so that when the device is screwed into opposing bone surfaces and coupled, the opposing pitch of the threads in the bone prevents the device from unscrewing.

The embodiments described herein are not limited to any particular pitch of one rotation of the continuous spiraling thread. For example, the pitch may be 5 mm or greater, 4 mm, 3 mm, 2 mm, 1 mm, or any distance in between the aforementioned distances.

Connector 2140, as shown in FIG. 21, may comprise a plurality of notches 2142 for interlocking with a reversibly engaging locking mechanism of female component 2120. The reversibly engaging locking mechanism may include a ring that can expand to allow insertion of connector 2140. After passing the ring, the notches 2142 may engage with the reversibly engaging locking mechanism which locks connector 2140 of the male component 2130 within the female component 2120 such that the bone pieces that the male component 2130 and the female component 2120 are screwed into become coupled or fused as a bone fusion. Removal of connector 2140 from female component 2120 is facilitated by the ring of the reversibly engaging locking mechanism which acts as a friction axial lock which responds to a predetermined amount of longitudinal force applied to connector 2140 which causes connector 2140 to be removeable or adjustable from within cavity 2129.

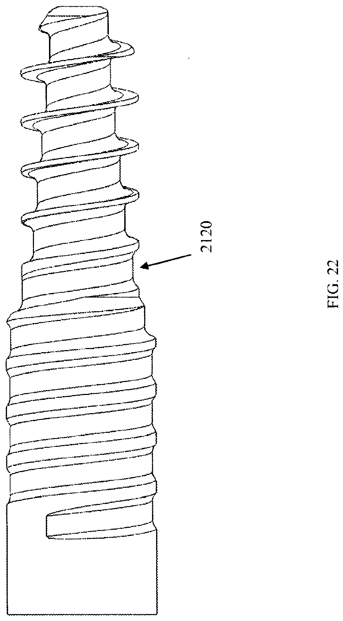

FIG. 22 illustrates a female component of a bone fusion device in accordance with an embodiment of the present disclosure. FIG. 22 depicts female component 2120 of an embodiment of the bone fusion device.

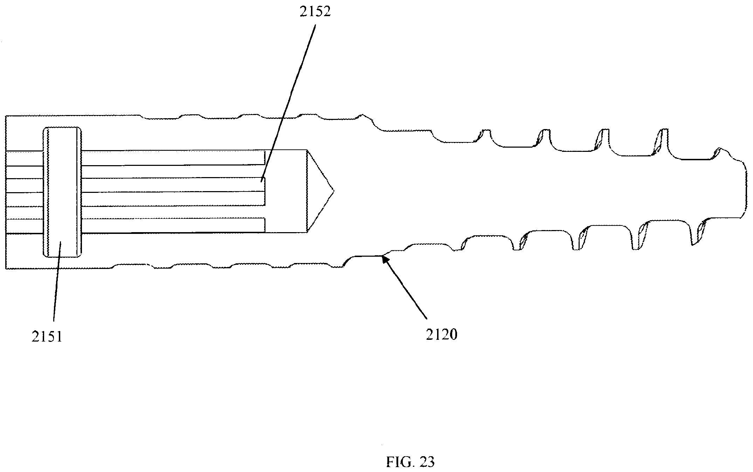

FIG. 23 illustrates a cross-sectional view of a female component of a bone fusion device in accordance with an embodiment of the present disclosure. Female component 2120 as illustrated, includes a reversibly engaging locking mechanism. Reversibly engaging locking mechanism resides within cavity 2129 and includes a ring 2151 and a plurality of grooves 2152 for receiving connector 2140. Ring 2151 acts as a friction axial lock which can interact with the notches of connector 2140. This facilitates incremental insertion of connector 2140. Similarly, ring 2151 also supports incremental removal or adjustment of connector 2140. Ring 2151 itself may comprise one or more ridges for catching or engaging with the notches of connector 2140 as connector 2140 is inserted or removed.

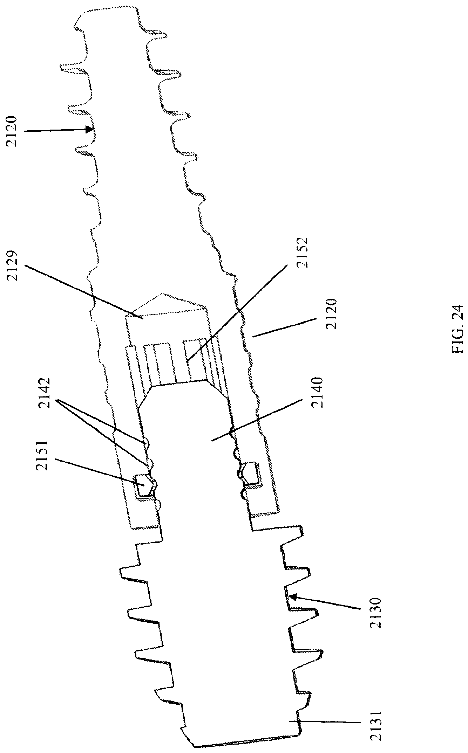

FIG. 24 illustrates a cross-sectional view of a bone fusion device where the male component has been inserted into the female component, in accordance with an embodiment of the present disclosure. As shown, connector 2140 of male component 2130 has been inserted into cavity 2129 of female component 2120. The connector 2140 has engaged with grooves 2152 of the reversibly engaging locking mechanism. Additionally, the notches 2142 of connector 2140 have engaged with ring 2151 and are thus locked in place. Connector 2140 may be inserted longitudinally such that the notches 2142 engage with ring 2151 which acts as a friction axial lock. Increasing amounts of compressive force applied to end 2131 of male component 2130 causes connector 2140 to be implanted deeper within female component 2120. A greater amount of longitudinal force applied outwards would cause removal or outwards movement of connector 2140 in a direction outwards from cavity 2129 in which connector 2140 may be implanted.

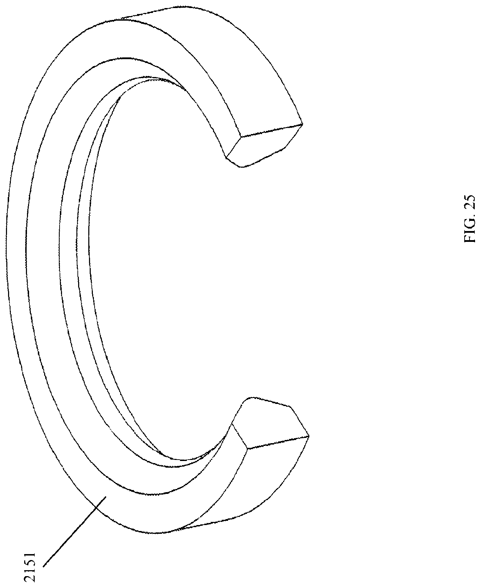

FIG. 25 illustrates an elevated magnified perspective view illustrating a ring, in accordance with an embodiment of the present disclosure. Ring 2151 is a partial circle with a protruding ridge around its interior surface for engaging the notches of the connector 2140 of FIG. 24. Ring 2151 may be inserted into the cavity 2129 of the female component 2120 at its top 2122, or at an opening in the wall of the female component 2120 similar to the opening shown in FIG. 10.

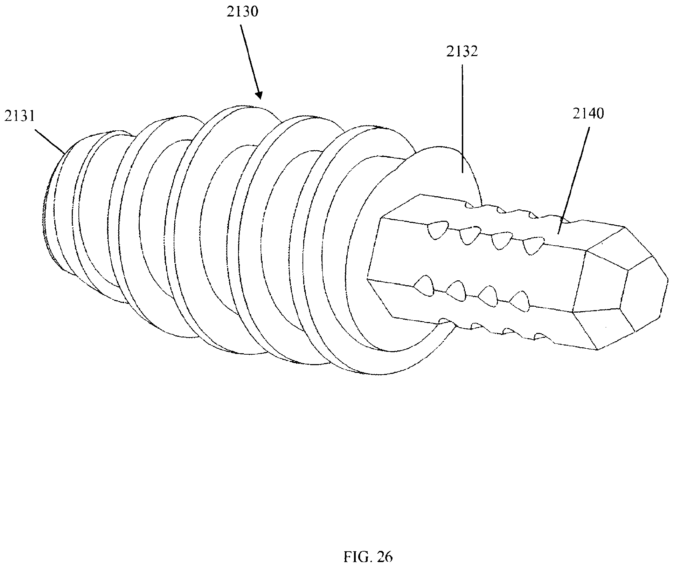

FIG. 26 illustrates an elevated side view perspective illustrating a male component, in accordance with an embodiment of the present disclosure. Connector 2140 is hexagonal, with notches for engaging the ring 2151 of FIG. 25, which supports adjustment within, or removal from, female component 2120. Connector 2140's hexagonal shape further provides rotational stability of the male and female components, 2130, 2120, with respect to each other when coupled via the connector 2140.

The foregoing Detailed Description is understood as being in every respect illustrative and exemplary, but not restrictive, and the scope of the embodiments of the present disclosure disclosed herein is not to be determined from the Detailed Description, but rather from the claims as interpreted according to the full breadth permitted by the patent laws. It is understood that the embodiments shown and described herein are only illustrative of the principles of the present disclosure and that various modifications may be implemented by those skilled in the art without departing from the scope and spirit of the present disclosure. Those skilled in the art could implement various other feature combinations without departing from the scope and spirit of the present disclosure. The scope of the present invention is only defined by the appended claims.

## Claims

1. A reversible bone coupling device (10) comprising:
a first component (20) comprising:
a first elongated stem portion for insertion from a first end (21) longitudinally into a surface of a first bone piece ; and
a second component (30) comprising:
a second elongated stem portion for insertion from a second end (31) longitudinally into a surface of a second bone piece, and
a connector (40) extending from a second top (32) and comprising a plurality of grooves (41) for coupling with the first component (20) and locking therein, wherein the first component (20) comprises a cavity (29) that extends within the first elongated stem portion from a first top (22) toward the first end (21), the cavity (29) capable of receiving the connector (40) of the second component (30), and
wherein the first component (20) includes a reversibly engaging locking mechanism (50)
**characterised in that** the cavity (29) includes an inner surface with a plurality of lobes (51), and **in that** the locking mechanism (50) comprises an internal lock ring (52) positioned within the cavity (29) of the first component (20) for selectively engaging and disengaging the connector (40) relative to the first component (20), the internal lock ring (52) including outwardly-facing bosses (53) positioned within the lobes (51) of the cavity (29) and an inwardly-facing chamfer (54) for engaging the grooves (41) of the connector (40).

2. The device (10) of claim 1, wherein the reversibly engaging locking mechanism (50) supports incremental locking of the connector (40) of the second component (30) with the first component (20).

3. The device (10) of claim 1, wherein the reversibly engaging locking mechanism (50) supports incremental disengagement of the connector (40) of the second component (30) from the first component (20).

4. The device (10) of claim 1, wherein each groove (41) is adapted for interlocking with the chamfer (54) of the internal lock ring (52).

5. The device (10) of claim 1, wherein the device (10) promotes fusion of the first bone piece to the second bone piece.

6. The device (10) of claim 1, wherein the chamfer (54) of the internal lock ring (52) is integral and configured to mate with any of a plurality of grooves (41) of the connector (40).

7. The device (10) of claim 6, wherein the chamfer (54) and bosses (53) of the internal lock ring (52) are integral.

8. The device (10) of claim 1, wherein the bosses (53) of the internal lock ring (52) prevent rotation of the internal lock ring (52) within the cavity (29).

9. The device (10) of claim 1, wherein the connector (40) is capable of being adjustably positioned in relation to the second top (32) when coupling with the first component (20).

10. The device (10) of claim 1, wherein each of the first elongated stem portion and the second elongated stem portion are at least one of cylindrical and conical.

11. The device (10) of claim 1, wherein at least one of the first elongated stem portion and the second elongated stem portion is a spiraling thread (24).

12. The device (10) of claim 11, wherein the spiraling thread (24) is a continuous spiraling thread (24).

13. The device (10) of claim 1, wherein the internal lock ring (52) is a C-shape with the bosses (53) being positioned at the ends of the C-shape.

14. The device (10) of claim 13, wherein the chamfer (54) of the internal lock ring (52) is positioned substantially midway between the bosses (53).

15. The device (10) of claim 1, wherein the chamfer (54) protrudes into the cavity (29) such that when the connector (40) is inserted into the cavity (29), the chamfer (54) engages with grooves (41) of the connector (40) to selectively lock the second component (30) with the first component (20).

16. The device (10) of claim 1, wherein the second elongated stem portion and connector (40) are integral and orientated at a fixed angle.

## Patentansprüche

1. Reversible Knochenkupplungsvorrichtung (10), umfassend:
eine erste Komponente (20), umfassend:
einen ersten länglichen Schaftteil zum Einführen von einem ersten Ende (21) aus längs in eine Oberfläche eines ersten Knochenstücks; und
eine zweite Komponente (30), umfassend:
einen zweiten länglichen Schaftteil zum Einführen von einem zweiten Ende (31) aus längs in eine Oberfläche eines zweiten Knochenstücks und
einen sich von einer zweiten Oberseite (32) erstreckenden Verbinder (40), umfassend eine Mehrzahl von Vertiefungen (41), zum Kuppeln mit der ersten Komponente (20) und Einrasten in derselben,
wobei die erste Komponente (20) einen Hohlraum (29) umfasst, der sich innerhalb des ersten länglichen Schaftteils von einer ersten Oberseite (22) zu dem ersten Ende (21) hin erstreckt, wobei der Hohlraum (29) zum Aufnehmen des Verbinders (40) der zweiten Komponente (30) in der Lage ist und
wobei die erste Komponente (20) einen reversibel eingreifenden Einrastmechanismus (50) einschließt,
**dadurch gekennzeichnet, dass** der Hohlraum (29) eine innere Oberfläche mit einer Mehrzahl von Vorsprüngen (51) einschließt und dadurch, dass der Einrastmechanismus (50) einen innen liegenden Einrastring (52) aufweist, der innerhalb des Hohlraums (29) der ersten Komponente (20) positioniert ist, um den Verbinder (40) relativ zu der ersten Komponente (20) selektiv in Eingriff zu nehmen und außer Eingriff zu bringen, wobei der innen liegende Einrastring (52) nach außen weisende runde Vorsprünge (53) einschließt, die innerhalb der Vorsprünge (51) des Hohlraums (29) positioniert sind, sowie eine nach innen weisenden Abfasung (54), um die Vertiefungen (41) des Verbinders (40) in Eingriff zu nehmen.

2. Vorrichtung (10) nach Anspruch 1, wobei der reversibel eingreifende Einrastmechanismus (50) ein inkrementelles Verrasten des Verbinders (40) der zweiten Komponente (30) mit der ersten Komponente (20) unterstützt.

3. Vorrichtung (10) nach Anspruch 1, wobei der reversibel eingreifende Einrastmechanismus (50) ein inkrementelles Außer-Eingriff-Bringen des Verbinders (40) der zweiten Komponente (30) aus der ersten Komponente (20) unterstützt.

4. Vorrichtung (10) nach Anspruch 1, wobei jede Vertiefung (41) geeignet ist, mit der Abfasung (54) des innen liegenden Einrastrings (52) zu verrasten.

5. Vorrichtung (10) nach Anspruch 1, wobei die Vorrichtung (10) die Fusion des ersten Knochenstücks an dem zweiten Knochenstück fördert.

6. Vorrichtung (10) nach Anspruch 1, wobei die Abfasung (54) des innen liegenden Einrastrings (52) integral und ausgeführt ist, sich durch Ineingriffnahme mit einer von einer Vielzahl von Vertiefungen (41) des Verbinders (40) zu verbinden.

7. Vorrichtung (10) nach Anspruch 6, wobei die Abfasung (54) und runden Vorsprünge (53) des innen liegenden Einrastrings (52) integral sind.

8. Vorrichtung (10) nach Anspruch 1, wobei die runden Vorsprünge (53) des innen liegenden Einrastrings (52) die Rotation des innen liegenden Einrastrings (52) innerhalb des Hohlraums (29) verhindern.

9. Vorrichtung (10) nach Anspruch 1, wobei der Verbinder (40) in der Lage ist, beim Kuppeln mit der ersten Komponente (20) in Bezug auf die zweite Oberseite (32) verstellbar positioniert zu werden.

10. Vorrichtung (10) nach Anspruch 1, wobei der erste längliche Schaftteil und der zweite längliche Schaftteil jeweils zumindest eines von zylindrisch und konisch sind.

11. Vorrichtung (10) nach Anspruch 1, wobei zumindest einer von dem ersten länglichen Schaftteil und dem zweiten länglichen Schaftteil ein Spiralgewinde (24) ist.

12. Vorrichtung (10) nach Anspruch 11, wobei das Spiralgewinde (24) ein durchgehendes Spiralgewinde (24) ist.

13. Vorrichtung (10) nach Anspruch 1, wobei der innen liegende Einrastring (52) eine C-Form ist und die runden Vorsprünge (53) an den Enden der C-Form positioniert sind.

14. Vorrichtung (10) nach Anspruch 13, wobei die Abfasung (54) des innen liegenden Einrastrings (52) im Wesentlichen mittig zwischen den runden Vorsprüngen (53) positioniert ist.

15. Vorrichtung (10) nach Anspruch 1, wobei die Abfasung (54) in den Hohlraum (29) hineinragt, sodass bei Einführen des Verbinders (40) in den Hohlraum (29) die Abfasung (54) Vertiefungen (41) des Verbinders (40) in Eingriff nimmt, um die zweite Komponente (30) selektiv mit der ersten Komponente (20) zu verrasten.

16. Vorrichtung (10) nach Anspruch 1, wobei der zweite längliche Schaftteil und Verbinder (40) integral und in einem festen Winkel ausgerichtet sind.

## Revendications

1. Dispositif de couplage osseux réversible (10) comprenant :
un premier composant (20) comprenant :
une première partie de tige allongée destinée à être insérée à partir d'une première extrémité (21) longitudinalement dans une surface d'une première pièce d'os ; et
un second composant (30) comprenant :
une seconde partie de tige allongée destinée à être insérée à partir d'une seconde extrémité (31) longitudinalement dans une surface d'une seconde pièce d'os, et
un connecteur (40) s'étendant à partir d'une seconde partie supérieure (32) et comprenant une pluralité de rainures (41) pour se coupler avec le premier composant (20) et se verrouiller à l'intérieur,
dans lequel le premier composant (20) comprend une cavité (29) qui s'étend à l'intérieur de la première partie de tige allongée à partir d'une première partie supérieure (22) vers la première extrémité (21), la cavité (29) pouvant recevoir le connecteur (40) du second composant (30), et
dans lequel le premier composant (20) comprend un mécanisme de verrouillage de mise en prise réversible (50), **caractérisé en ce que** la cavité (29) comprend une surface interne avec une pluralité de lobes (51) et **en ce que** le mécanisme de verrouillage (50) comprend une bague de verrouillage interne (52) positionnée à l'intérieur de la cavité (29) du premier composant (20) pour mettre en prise et dégager sélectivement le connecteur (40) par rapport au premier composant (20), la bague de verrouillage interne (52) comprenant des bossages (53) orientés vers l'extérieur positionnés à l'intérieur des lobes (51) de la cavité (29) et un chanfrein (54) orienté vers l'intérieur pour mettre en prise les rainures (41) du connecteur (40).

2. Dispositif (10) selon la revendication 1, dans lequel le mécanisme de verrouillage de mise en prise réversible (50) supporte le verrouillage incrémentiel du connecteur (40) du second composant (30) avec le premier composant (20).

3. Dispositif (10) selon la revendication 1, dans lequel le mécanisme de verrouillage de mise en prise réversible (50) supporte le dégagement incrémentiel du connecteur (40) du second composant (30) du premier composant (20) .

4. Dispositif (10) selon la revendication 1, dans lequel chaque rainure (41) est adaptée pour s'emboîter avec le chanfrein (54) de la bague de verrouillage interne (52).

5. Dispositif (10) selon la revendication 1, dans lequel le dispositif (10) favorise la fusion de la première pièce d'os par rapport à la seconde pièce d'os.

6. Dispositif (10) selon la revendication 1, dans lequel le chanfrein (54) de la bague de verrouillage interne (52) est solidaire et configuré pour se coupler avec l'une quelconque d'une pluralité de rainures (41) du connecteur (40) .

7. Dispositif (10) selon la revendication 6, dans lequel le chanfrein (54) et les bossages (53) de la bague de verrouillage interne (52) sont solidaires.

8. Dispositif (10) selon la revendication 1, dans lequel les bossages (53) de la bague de verrouillage interne (52) empêchent la rotation de la bague de verrouillage interne (52) à l'intérieur de la cavité (29).

9. Dispositif (10) selon la revendication 1, dans lequel le connecteur (40) peut être positionné de manière ajustable par rapport à la seconde partie supérieure (32) lors du couplage avec le premier composant (20).

10. Dispositif (10) selon la revendication 1, dans lequel chacune parmi la première partie de tige allongée et la seconde partie de tige allongée est au moins l'un parmi un cylindre et un cône.

11. Dispositif (10) selon la revendication 1, dans lequel au moins l'une parmi la première partie de tige allongée et la seconde partie de tige allongée est un filetage en spirale (24).

12. Dispositif (10) selon la revendication 11, dans lequel le filetage en spirale (24) est un filetage en spirale (24) continu.

13. Dispositif (10) selon la revendication 1, dans lequel le bague de verrouillage interne (52) est une forme de C avec les bossages (53) qui sont positionnés au niveau des extrémités de la forme de C.

14. Dispositif (10) selon la revendication 13, dans lequel le chanfrein (54) de la bague de verrouillage interne (52) est positionné sensiblement à mi-chemin entre les bossages (53).

15. Dispositif (10) selon la revendication 1, dans lequel le chanfrein (54) fait saillie dans la cavité (29) de sorte que lorsque le connecteur (40) est inséré dans la cavité (29), le chanfrein (54) se met en prise avec les rainures (41) du connecteur (40) pour verrouiller sélectivement le second composant (30) avec le premier composant (20).

16. Dispositif (10) selon la revendication 1, dans lequel la seconde partie de tige allongée et le connecteur (40) sont solidaires et orientés à un angle fixe.
